# EUROPÄISCHE PATENTANMELDUNG

(11) **EP 3 045 153 A1**
(43) Veröffentlichungstag der Anmeldung: **20.07.2016**
(21) Anmeldenummer: 15151002.1
(22) Anmeldetag: 13.01.2015
(51) Int. Cl.: A61F 5/448, A61F 5/443

(54) **Einrichtung zum Aufsetzen auf eine künstliche Körperöffnung eines Patienten**

(71) Anmelder: Scheurer, Alfred, 3706 Leissigen (CH)
(72) Erfinder: Scheurer, Alfred, 3706 Leissigen (CH); Scheurer, Verena, 3706 Leissigen (CH)
(74) Vertreter: BOVARD AG

(57) **Zusammenfassung**

Eine Einrichtung zum Aufsetzen auf eine künstliche Körperöffnung (2) eines Patienten umfasst ein plattenförmiges Element (3) welches mit ersten Kupplungsmitteln (7) ausgestattet ist, auf welche ein Auffangbeutel (8) aufsetzbar ist, der entsprechende zweite Kupplungsmittel (10) aufweist. Das plattenförmige Element (3) ist an einer Oberfläche (4) mit Haftmitteln (5) versehen, wodurch das plattenförmige Element (3) auf die die künstliche Körperöffnung (2) umgebende Hautoberfläche (1) des Patienten aufklebbar ist. Auf das plattenförmige Element (3) ist eine dehnbare Abdeckfolie (11) aufsetzbar, welche das plattenförmige Element (3) allseitig überragt, und welche mit einer zentralen Ausnehmung (13) ausgestattet ist, durch welche die ersten Kupplungsmittel (7) im auf das plattenförmige Element (3) aufgesetzten Zustand hindurchragen. Die Abdeckfolie (11) ist einseitig mit einer Haftschicht (12) versehen, die auf der Hautoberfläche (1) und dem plattenförmigen Element (3) lösbar haftet. Durch diese Abdeckfolie (11) wird die Dichtheit dieser Einrichtung in optimaler Weise verbessert.

## Beschreibung

Die vorliegende Erfindung bezieht sich auf eine Einrichtung zum Aufsetzen auf eine künstliche Körperöffnung eines Patienten, umfassend ein plattenförmiges Element, welches zentral mit einer ersten Öffnung versehen ist, welche erste Öffnung erste Kupplungsmittel aufweist, auf welche ein Auffangbeutel aufsetzbar ist, der eine entsprechende zweite Öffnung mit zweiten Kupplungsmitteln aufweist, sodass der Auffangbeutel dicht mit dem plattenförmigen Element lösbar verbindbar ist, und die eine Oberfläche des plattenförmigen Elementes mit Haftmitteln versehen ist und dadurch das plattenförmige Element auf die die künstliche Körperöffnung umgebende Hautoberfläche des Patienten aufklebbar ist.

Derartige Einrichtungen sind in vielfältiger Weise bekannt. Derartige Einrichtungen dienen dazu, aus der jeweiligen künstlichen Körperöffnung, beispielsweise einem künstlichen Darmausgang, austretende Körperflüssigkeiten und/oder Körperausscheidungen aufzufangen und im Auffangbeutel zu sammeln. Hierzu wird das plattenförmige Element, das einseitig mit Haftmitteln versehen ist, auf die die künstliche Körperöffnung umgebende Hautoberfläche des Patienten aufgeklebt, sodass die künstliche Öffnung durch die im plattenförmigen Element angebrachte Öffnung hindurchragt. Auf dieses plattenförmige Element wird der Auffangbeutel aufgesetzt, wozu entsprechende Kupplungsmittel vorgesehen sind, sodass zwischen Auffangbeutel und plattenförmigem Element eine dichte Verbindung gebildet wird.

Die Haftverbindung zwischen diesem plattenförmigen Element und der Hautoberfläche des Patienten, auf welche dieses plattenförmige Element aufgeklebt ist, gewährleistet oftmals nicht die gewünschte Dichtheit. Undichtheiten oder Leckagen können beispielsweise durch Hautfalten entstehen, sie können aber auch durch Bewegungen des Patienten entstehen, sodass die aus der künstlichen Körperöffnung austretende Körperflüssigkeit zwischen dem plattenförmigen Element und der Hautoberfläche sich einen Weg bahnen und austreten kann.

Eine derartige Leckage ist für den jeweiligen Patienten äusserst unangenehm. Insbesondere ist dieser Patient verunsichert, da praktisch nicht vorhersehbar ist, ob nun das plattenförmige Element, das auf die Hautoberfläche aufgeklebt ist, wirklich sicher ist und keine Leckage auftreten kann oder ob dies eben passieren kann.

Um eine Verbesserung der Haftfähigkeit des plattenförmigen Elementes auf der Hautoberfläche des Patienten erreichen zu können und damit eine bessere Abdichtung erhalten wird, werden zusätzlich Klebepasten oder Kleberinge eingesetzt, die rund um die künstliche Körperöffnung angebracht werden, auf welche dann das plattenförmige Element aufgesetzt wird. Mit derartigen Mitteln kann zwar in gewissen Fällen eine verbesserte Abdichtung erreicht werden, Leckagen treten aber immer noch relativ häufig auf.

Die Aufgabe der vorliegenden Erfindung besteht somit darin, die Einrichtung zum Aufsetzen auf eine künstliche Körperöffnung eines Patienten derart zu verbessern, dass die Dichtheit zwischen dieser Einrichtung und der Hautoberfläche des Patienten um die künstliche Körperöffnung praktisch gewährleistet ist, wodurch die Bewegungseinschränkung des Patienten möglichst ausgeräumt wird und das Sicherheitsgefühl zurückgegeben wird.

Erfindungsgemäss erfolgt die Lösung dieser Aufgabe dadurch, dass auf das plattenförmige Element eine dehnbare Abdeckfolie aufsetzbar ist, welche das plattenförmige Element allseitig überragt, welche mit einer zentralen Ausnehmung ausgestattet ist, durch welche die ersten Kupplungsmittel im auf das plattenförmige Element aufgesetzten Zustand hindurchragen, und welche Abdeckfolie einseitig mit einer Haftschicht versehen ist, die auf der Hautoberfläche und dem plattenförmigen Element lösbar haftet.

Mit dieser erfindungsgemässen Lösung wird erreicht, dass Leckagen im Bereich von künstliche Körperöffnungen praktisch vermieden werden können, die elastische und dehnbare Abdeckfolie passt sich der Hautoberfläche des Patienten in optimaler Weise an, durch die Dehnbarkeit und die Elastizität der Abdeckfolie ist auch ein optimaler Tragkomfort gewährleistet. Eine derartig ausgestaltete Abdeckfolie kann zudem für viele derartig eingesetzte Einrichtungen verwendet werden, sie kann auch entsprechend an eingesetzte Einrichtungen angepasst werden.

Mit einer derartig verbesserten Einrichtung, die eine Dichtheit gewährleistet und Leckagen praktisch ausschliessen kann, erhält der Patient die gewünschte Sicherheit, seine Bewegungsfreiheit wird massiv verbessert, eine Berufstätigkeit kann wieder ausgeübt werden, ebenso Sportaktivitäten. Dem Patienten wird ermöglicht, ein nahezu normales Leben zu führen mit einer um ein Vielfaches gesteigerten Lebensqualität.

In vorteilhafter Weise ist die Abdeckfolie aus Polyurethan gebildet, wodurch die gewünscht Elastizität und Dehnbarkeit unter Gewährleistung der erforderlichen Festigkeit erreicht werden kann.

In vorteilhafter Weise ist die Haftschicht aus einem Polyacrylatklebstoff gebildet, der einerseits die gewünschte Haftkraft bietet und andererseits sehr hautverträglich ist.

In vorteilhafter Weise ist auf die Haftschicht der Abdeckfolie eine Abdeckung aufsetzbar, die diese schützt, welche Abdeckung zum Aufkleben der Abdeckfolie auf das plattenförmige Element und die Hautoberfläche entfernbar ist. Dadurch kann beispielsweise die Abdeckfolie in optimaler Weise verpackt und mitgeführt werden, ohne dass ein unerwünschtes Verkleben mit anderen Gegenständen auftreten kann.

Eine weitere vorteilhafte Ausgestaltung der Erfindung besteht darin, dass die Abdeckung aus einem ersten Blatt, welches einen mittleren Streifen der Abdeckfolie abdeckt, und aus zwei Blattstreifen besteht, welche die beiden einander gegenüberliegenden Seitenstreifen der Abdeckfolie abdeckt. Hierdurch kann die Abdeckfolie in optimaler Weise aufgeklebt werden, zum Aufkleben wird als erste Blatt, welches den mittleren Streifen der Abdeckfolie abdeckt, weggenommen, die Abdeckfolie kann an den zwei Blattstreifen festgehalten werden, die die Seitenstreifen der Abdeckfolie abdecken, der mittlere Bereich der Abdeckfolie kann auf das plattenförmige Element und die umgebende Hautoberfläche des Patienten aufgeklebt werden, danach können die beiden Blattstreifen abgezogen werden, die Seitenbereiche der Abdeckfolie können dann an die entsprechenden Stellen angedrückt werden.

In vorteilhafter Weise überragt die Abdeckfolie das plattenförmige Element allseitig um einige Zentimeter, wodurch eine optimale Dichtigkeit der Einrichtung gewährleistet ist. Es hat sich gezeigt, dass es optimal ist, wenn die Abdeckfolie das plattenförmige Element allseitig um etwa drei Zentimeter überragt.

In vorteilhafter Weise beträgt die Dicke der Abdeckfolie einige Zehntelsmillimeter, dadurch wird die gewünschte Dehnbarkeit und Elastizität mit der erforderlichen Festigkeit erhalten.

Eine Ausführung der Erfindung wird nachfolgend anhand der beiliegenden Zeichnung beispielhaft näher erläutert.

Es zeigt
Figur 1 im Schnitt und in schematischer Darstellung eine Ausführungsform einer erfindungsgemässen Einrichtung im auf eine künstliche Körperöffnung aufgesetzten Zustand eines Patienten;
Figur 2 eine Draufsicht auf das plattenförmige Element;
Figur 3a eine Draufsicht auf eine Abdeckfolie;
Figur 3b eine Schnittdarstellung der Abdeckfolie gemäss Figur 3a mit den beiden noch aufgesetzten Blattstreifen;
Figur 4 eine Draufsicht auf das plattenförmigen Element mit aufgesetzter Abdeckfolie; und
Figur 5 eine Draufsicht auf die erfindungsgemässe Einrichtung mit aufgesetzten Auffandbeutel.

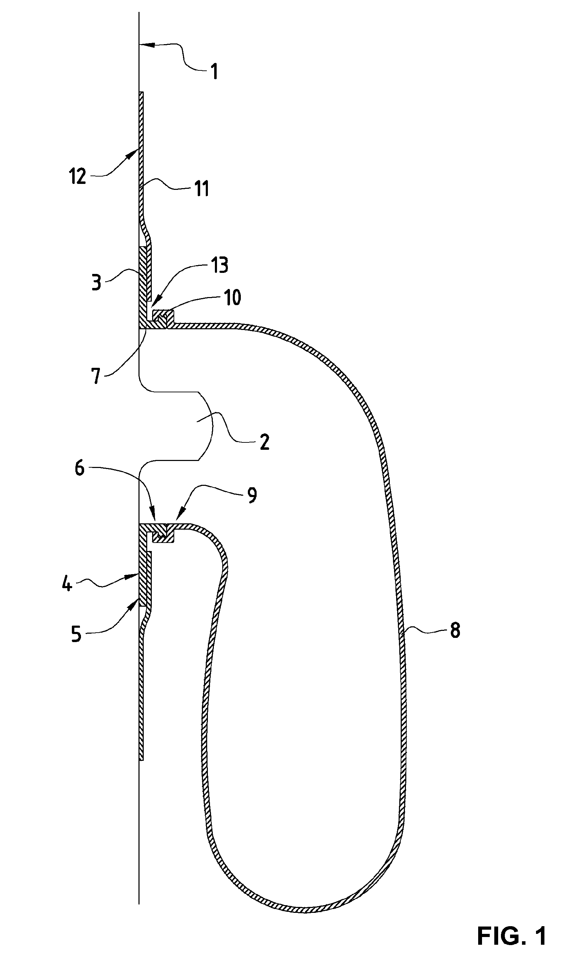

Figur 1 zeigt in sehr schematischer Darstellung die Hautoberfläche 1 eines Patienten mit einer künstlichen Körperöffnung 2. Auf die Hautoberfläche 1 und die künstliche Körperöffnung 2 ist ein plattenförmiges Element 3 aufgesetzt. Dieses plattenförmige Element 3 ist an der der Hautoberfläche 1 zugewandten Oberfläche in bekannter Weise mit Haftmitteln 5 versehen. Diese Haftmittel 5 können eine Schicht aus Klebemittel sein, mit welcher die Oberfläche 4 des plattenförmigen Elementes 3 beschichtet ist, wobei das Klebemittel in bekannter Weise äusserst hautfreundlich ist, diese Haftmittel 5 können aber auch zusätzlich eine weiche Schicht umfassen, die beispielsweise aus einem schaumstoffähnlichen Material gebildet ist, welche Schicht beidseitig mit Haftmitteln versehen ist, und welche einerseits auf die Hautoberfläche 1 und andererseits auf die Oberfläche 4 des plattenförmigen Elementes 3 aufgesetzt wird. Neben der optimalen Hautverträglichkeit sollen die Haftmittel 5 eine möglichst gute Verbindung zwischen plattenförmigen Element 3 und Hautoberfläche 1 bieten.

Im plattenförmigen Element 3 zentral angeordnet ist eine erste Öffnung 6. Diese erste Öffnung 6 lässt Platz für die künstliche Körperöffnung 2. An dieser ersten Öffnung 6 angeordnet sind erste Kupplungsmittel 7. Auf diese ersten Kupplungsmittel 7 ist ein Auffangbeutel 8 aufsetzbar, welcher mit einer zweiten Öffnung 9 mit zweiten Kupplungsmitteln 10 versehen ist. Über diese zweiten Kupplungsmittel 10 ist der Auffangbeutel 8 in bekannter Weise mit den ersten Kupplungsmitteln 7 des plattenförmigen Elementes 3 lösbar verbindbar, der Auffangbeutel 8 ist dadurch dicht am plattenförmigen Element 3 angekoppelt. Hierbei sind verschiedenste Lösungsmöglichkeiten bekannt, wie der Auffangbeutel 8 mit dem plattenförmigen Element 3 verbunden werden kann. Wie bereits vorgängig beschrieben worden ist, besteht ein gewisses Risiko, dass zwischen dem plattenförmigen Element 3 und der Hautoberfläche 1, auf welche dieses plattenförmige Element um die künstliche Körperöffnung 2 aufgeklebt ist, Undichtheiten auftreten können, durch welche aus der künstlichen Körperöffnung 2 austretende Körperflüssigkeit bzw. Körperausscheidungen austreten können, was für den Patienten sehr unangenehm ist. Um die hier austretenden Flüssigkeiten auffangen und zurückhalten zu können, ist auf das plattenförmige Element 3 eine Abdeckfolie 11 aufgesetzt. Diese Abdeckfolie 11, die später noch im Detail beschrieben wird, besitzt eine zentrale Ausnehmung 13, durch welche die ersten Kupplungsmittel 7 des plattenförmigen Elementes 3 hindurchragen können. Diese Abdeckfolie 11 ist einseitig mit einer Haftschicht 12 versehen. Diese Abdeckfolie 11 wird auf das plattenförmige Element 3 aufgeklebt, sodass der Randbereich, der die erste Öffnung 6 des plattenförmigen Elementes 3 umgibt, vollständig abgedeckt ist, die Abdeckfolie 11 überragt allseitig das plattenförmige Element 3, dieser Bereich haftet an der Hautoberfläche 1, welche das plattenförmige Element 3 umgibt. Durch diese derartig aufgeklebte Abdeckfolie 11 kann Körperflüssigkeit, die sich zwischen den plattenförmigen Element 3 und der Hautoberfläche 1 einen Weg hat bahnen können und aus dem plattenförmigen Element 3 austreten kann, zurückgehalten werden, die Dichtheit ist somit gewährleistet.

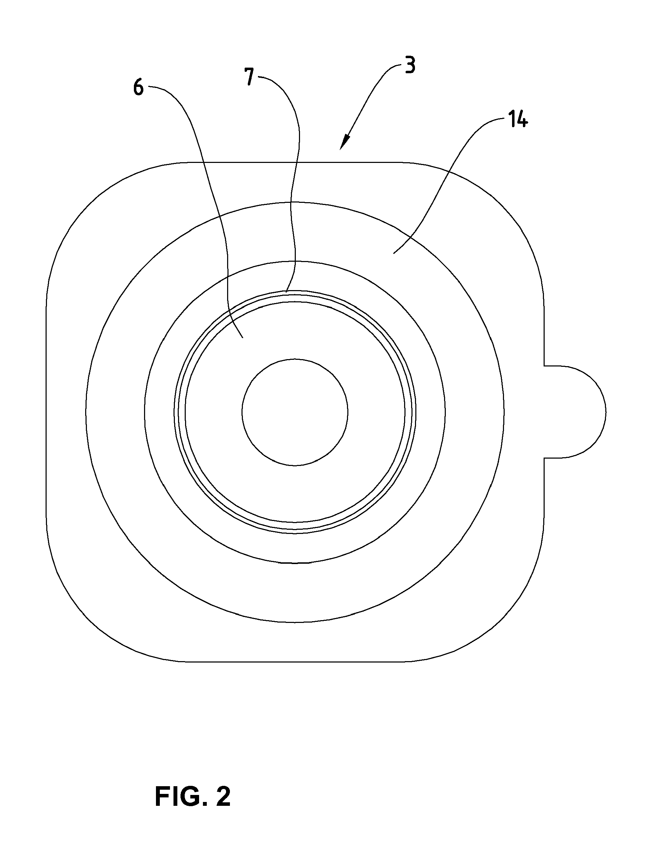

Figur 2 zeigt das plattenförmige Element 3 mit der ersten Öffnung 6 und den ersten Kupplungsmitteln 7. Wie bereits erwähnt worden ist, kann dieses plattenförmige Element 3 zusätzlich mit einer Haftschicht 14 ausgestattet sein.

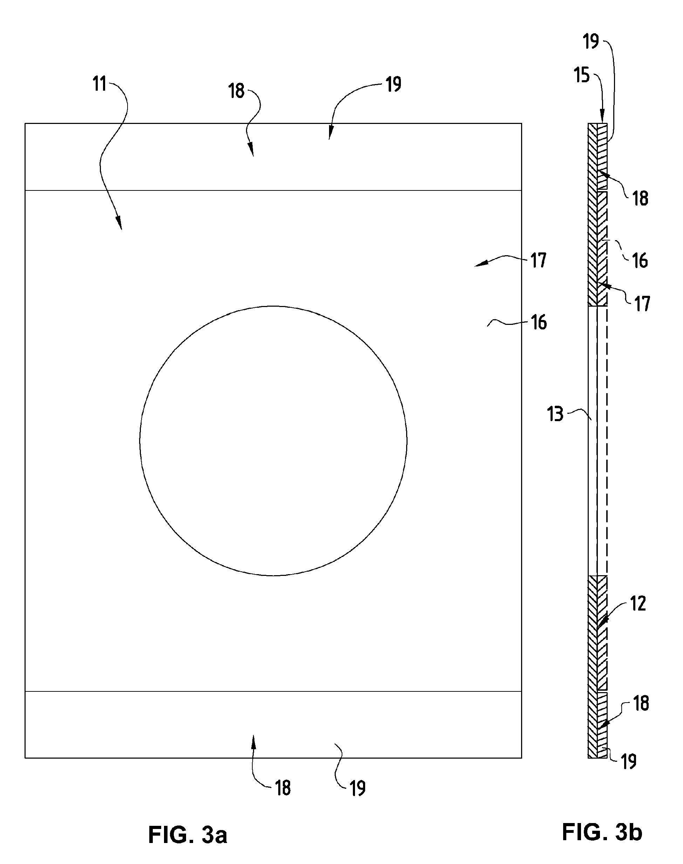

In den Figuren 3a und 3b ist die Abdeckfolie 11 dargestellt. Diese Abdeckfolie 11 ist mit der zentralen Ausnehmung 13 ausgestattet. Diese Abdeckfolie 11 ist einseitig mit der Haftschicht 12 ausgestattet. Diese Abdeckfolie kann aus Polyurethan gebildet sein, selbstverständlich sind auch andere geeignete Materialien einsetzbar. Die Haftschicht 12 besteht aus einem Polyacrylatklebstoff, auch hier sind andere geeignete Klebstoffe einsetzbar, die die erforderliche Hautverträglichkeit aufweisen. Die Abdeckfolie weist eine Dicke auf, die im Bereich von einigen Zehntelsmillimetern liegt, wodurch einerseits die gewünschte Elastizität und andererseits die erforderliche Festigkeit erreicht werden können.

Wie insbesondere aus Figur 3b ersichtlich ist, kann auf die Haftschicht 12 der Abdeckfolie 11 eine Abdeckung 15 aufgesetzt werden, wodurch diese Haftschicht 14 vor dem Gebrauch dieser Abdeckfolie 11 geschützt werden kann. Diese Abdeckung 15 kann aus einem beidseitig mit Polyethylen beschichteten und einseitig siliconisierten Papier gebildet sein.

Wie aus den Figuren 3a und 3b ersichtlich ist, kann diese Abdeckung 15 aus einem ersten Blatt 16 gebildet sein, welches einen mittleren Streifen 17 der Abdeckfolie 11 abdeckt. Die beiden einander gegenüberliegenden Seitenstreifen 18 der Abdeckfolie 11 können durch jeweils einen Blattstreifen 19 der Abdeckung 15 abgedeckt werden.

Zum Aufsetzen der Abdeckfolie 11 auf das plattenförmige Element 3 und die Hautoberfläche 1, wie dies in Figur 1 dargestellt ist, kann von der Haftschicht 12 der Abdeckfolie 11 das erste Blatt 16 abgezogen werden, das den mittleren Streifen 17 abdeckt. Die Abdeckfolie 11 kann an den beiden Blattstreifen 19 gehalten werden, die die beiden Seitenstreifen 18 der Haftschicht 12 der Abdeckfolie 11 abdecken, die Abdeckfolie kann dadurch auf das plattenförmige Element 3 und die Hautoberfläche 1 in optimaler Weise platziert und angepresst werden, danach können die beiden Blattstreifen 19 von der Haftschicht 12 abgezogen werden, die beiden Seitenstreifen 18 der Abdeckfolie 11 können danach ebenfalls an der Hautoberfläche 1 angeklebt werden. Die Abdeckfolie 11 kann dadurch in einfacher Weise auf das plattenförmige Element 3 und die Hautoberfläche 1 des Patienten aufgeklebt werden.

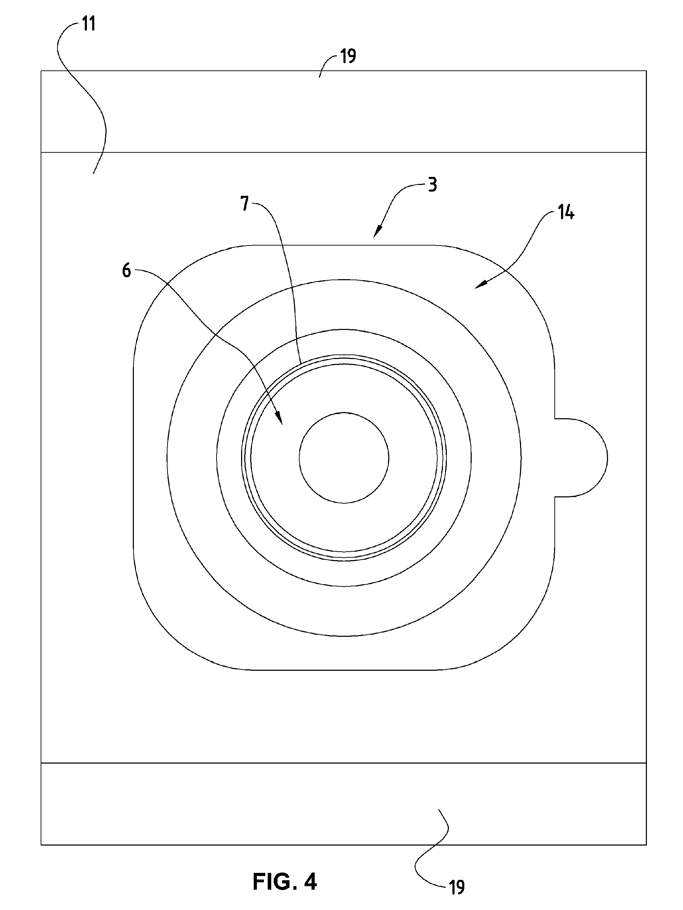

Aus Figur 4 ist das auf die Hautoberfläche 1 des Patienten aufgesetzte plattenförmige Element 3 ersichtlich, auf das plattenförmige Element 3 ist die Abdeckfolie 11 aufgesetzt, der hier dargestellte Zustand zeigt, dass die beiden Blattstreifen 19, die das Aufsetzen der Abdeckfolie 11 erleichtern, noch angebracht sind, diese beiden Blattstreifen 19 können nun entfernt werden, die Abdeckfolie 11 kann dann vollständig auf die Hautoberfläche 1 des Patienten aufgeklebt werden.

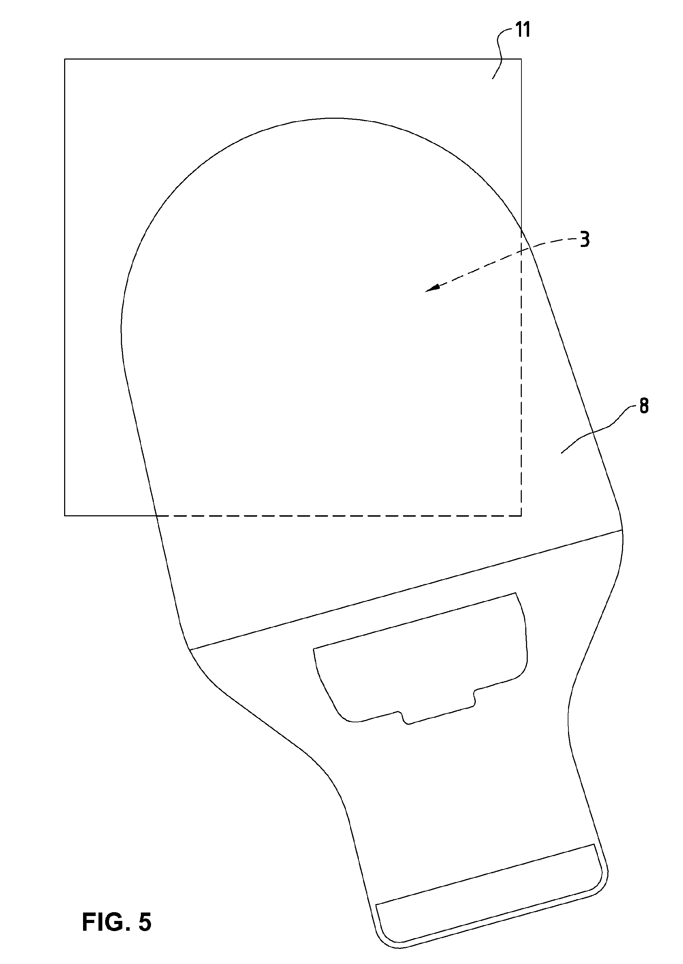

Wie aus Figur 5 ersichtlich ist, kann auf das plattenförmige Element 3, das auf die Hautoberfläche 1 des Patienten aufgesetzt ist und das durch die Abdeckfolie 11 abgedeckt ist, wie dies in Figur 4 dargestellt ist, in bekannter Weise der Auffangbeutel 8 aufgesetzt werden.

Mit dieser erfindungsgemässen Einrichtung kann erreicht werden, dass ein auf eine künstliche Körperöffnung eines Patienten anzubringendes plattenförmiges Element mit einer gewährleisteten Dichtheit aufgesetzt werden kann. Dies ergibt für den Patienten eine optimale Sicherheit, die Lebensqualität wird um ein Vielfaches gesteigert, der Patient kann ein praktisch normales Leben führen, insbesondere kann er wieder berufstätig werden, zudem ist ein praktisch unbehindertes Treiben von Sport wieder möglich.

## Patentansprüche

1. Einrichtung zum Aufsetzen auf eine künstliche Körperöffnung (2) eines Patienten, umfassend ein plattenförmiges Element (3), welches zentral mit einer ersten Öffnung (6) versehen ist, welche erste Öffnung (6) erste Kupplungsmittel (7) aufweist, auf welche ein Auffangbeutel (8) aufsetzbar ist, der eine entsprechende zweite Öffnung (9) mit zweiten Kupplungsmitteln (10) aufweist, so dass der Auffangbeutel (8) dicht mit dem plattenförmigen Element (3) lösbar verbindbar ist, und die eine Oberfläche (4) des plattenförmigen Elements (3) mit Haftmitteln (5) versehen ist und dadurch das plattenförmige Element (3) auf die die künstliche Körperöffnung (2) umgebende Hautoberfläche (1) des Patienten aufklebbar ist, **dadurch gekennzeichnet, dass** auf das plattenförmige Element (3) eine dehnbare Abdeckfolie (11) aufsetzbar ist, welche das plattenförmige Element (3) allseitig überragt, welche mit einer zentralen Ausnehmung (13) ausgestattet ist, durch welche die ersten Kupplungsmittel (7) im auf das plattenförmige Element (3) aufgesetzten Zustand hindurch ragen und welche einseitig mit einer Haftschicht (12) versehen ist, die auf der Hautoberfläche (1) und dem plattenförmigen Element (3) lösbar haftet.

2. Einrichtung nach Anspruch 1, **dadurch gekennzeichnet, dass** die Abdeckfolie (11) aus Polyurethan gebildet ist.

3. Einrichtung nach Anspruch 1 oder 2, **dadurch gekennzeichnet, dass** die Haftschicht (12) der Abdeckfolie (11) aus einem Polyacrylatklebstoff gebildet ist.

4. Einrichtung nach einem der Ansprüche 1 bis 3, **dadurch gekennzeichnet, dass** auf die Haftschicht (12) der Abdeckfolie (11) eine Abdeckung (15) aufsetzbar ist und diese schützt, welche Abdeckung (15) zum Aufkleben der Abdeckfolie (11) auf das plattenförmige Element (3) und die Hautoberfläche (1) entfernbar ist.

5. Einrichtung nach Anspruch 4, **dadurch gekennzeichnet, dass** die Abdeckung aus einem ersten Blatt (16), welches einen mittleren Streifen (17) der Abdeckfolie (11) abdeckt, und aus zwei Blattstreifen (19) besteht, welche die beiden einander gegenüberliegenden Seitenstreifen (18) der Abdeckfolie (11) abdeckt.

6. Einrichtung nach einem der Ansprüche 1 bis 5, **dadurch gekennzeichnet, dass** die Abdeckfolie (11) das plattenförmige Element (3) allseitig um einige Zentimeter überragt.

7. Einrichtung nach Anspruch 6, **dadurch gekennzeichnet, dass** die Abdeckfolie (11) das plattenförmige Element (3) allseitig um etwa drei Zentimeter überragt.

8. Einrichtung nach einem der Ansprüche 1 bis 7, **dadurch gekennzeichnet, dass** die Dicke der Abdeckfolie (11) einige Zehntelsmillimeter beträgt.
